# EUROPÄISCHE PATENTANMELDUNG

(11) **EP 0 759 556 A2**
(43) Veröffentlichungstag der Anmeldung: **26.02.1997**
(21) Anmeldenummer: 96110212.6
(22) Anmeldetag: 25.06.1996
(51) Int. Cl.: G01N 33/573, G01N 33/543, G01N 33/58

(54) **Verfahren zur Quantifizierung aktivierter Faktoren**

(30) Priorität: 24.07.1995 DE 19526700
(71) Anmelder: BEHRINGWERKE Aktiengesellschaft, 35001 Marburg (DE)
(72) Erfinder: Römisch, Jürgen, Dr., 35041 Marburg (DE); Stöhr, Hans-Arnold, 35083 Wetter (DE)

(57) **Zusammenfassung**

Die Erfindung betrifft sensitive Verfahren zur selektiven Bestimmung von Einzelfaktoren (aktivierten Gerinnungsproteasen) in z. B. Faktorenkonzentraten wie PPSB d. h. in Gemischen von aktivierten und nicht-aktivierten Faktoren.

## Beschreibung

Die Erfindung betrifft sensitive Verfahren zur selektiven Bestimmung von Einzelfaktoren (aktivierten Gerinnungsproteasen) in z. B. Faktorenkonzentraten wie PPSB d. h. in Gemischen von aktivierten und nicht-aktivierten Faktoren.

Angeborene und erworbene Mangelzustände an Plasmaproteinen oder Blutzellen werden heutzutage durch Infusion von Plasma, Zell-Konzentraten und aus Plasmapools gereinigten Proteinen und Faktorenkonzentraten substituiert. Neben der potentiellen Gefahr von Virusübertragungen, die durch Einzelspendertestung und entsprechende Virus-Abreicherungs- und - Inaktivierungsvertahren minimiert werden, werden unerwünschte Reaktionen, wie z.B. Blutdruckabfall, thrombogene oder blutungsfördernde Effekte, beobachtet. Diese Nebenwirkungen können durch die Anwesenheit sog. aktivierter Faktoren verursacht werden. Obwohl diese Präparate unter möglichst schonenden Bedingungen gereinigt werden und entsprechenden Qualitätskontrollen und Freigabekriterien unterliegen, wurden in Einzelfällen o.g. Nebenwirkungen berichtet. Analysen erhärteten retrospektiv häufig den Verdacht auf Vorliegen von aktivierten Faktoren in diesen Präparaten.

Die aus Blutplasma hergestellten Konzentrate können Spuren anderer Plasmaproteine enthalten, die üblicherweise die Effizienz und Sicherheit des Präparates nicht merklich beeinflussen. Liegen diese Proteine, u.a. Enzyme/Proteasen, in aktiviertem Zustand vor, kann dies nach Applikation fatale Folgen für den Patienten haben. Neben der aktivierten Form des Prothrombins, nämlich Thrombin, können auch Spuren der aktivierten Gerinnungsfaktoren VII,IX,X,XI,XII oder des Präkallikreins oder des Proteins C (F VIIa, F IXa, FXa, F XIa, F XIIa, Kallikrein, APC) oder des Komplementsystemes enthalten sein. Jedoch auch die gereinigten Proteine selbst, ob über herkömmliche Fällungs- und chromatographische Methoden aus Plasma oder anderen Ausgangsmaterialien oder rekombinant hergestellt, können partiell aktiviert vorliegen. Entsprechend wichtig ist die Detektion dieser aktivierten Faktoren. Da diese bereits in kleinsten Konzentrationen wirksam sind und auslösende bzw. verstärkende in-vivo Effekte verursachen können, sind möglichst sensitive und spezifische Tests erforderlich.

Derzeit etablierte Methoden erfassen z.B. die enzymatischen/proteolytischen Aktivitäten dieser Faktoren, wogegen deren Zymogene entsprechend inaktiv sind. Üblicherweise wird versucht, diese Aktivitäten mit Hilfe chromogener Substrate zu erfassen, wobei die amidolytische Freisetzung z.B. von p-Nitrophenylanilid oder fluorogenen Gruppen photometrisch erfaßt und ausgewertet wird. Obwohl diese Substrate für bestimmte Proteasen bzw. aktivierte Faktoren optimiert sind, werden sie in geringerem/größeren Ausmaß auch von anderen Proteasen gespalten. Besonders in komplexen Protein(Protease)-Gemischen, wie z.B. Prothrombin-Komplex-Konzentraten, fällt daher die Differenzierung und Quantifizierung einzelner Komponenten - und damit auch mehr oder weniger 'schädlicher' Aktivitäten- schwer. Dies wird erst dann möglich, wenn eine vergleichende Messung in Gegenwart eines sehr spezifischen Inhibitors durchgeführt wird, wie im Falle des Thrombins +/-Hirudin, mit dem die spezielle Aktivität ausgeblendet werden kann. Bei den anderen Proteasen ist dies bisher nicht möglich, da die verfügbaren Inhibitoren zwar teilweise sehr potent aber nicht ausreichend spezifisch sind.

Sehr selektive und sensitive Verfahren stellen immunchemische Nachweissysteme dar, wie z. B. RIA, ELISA etc. Geeignet wären entsprechende Testsysteme, die speziell die aktivierte Protease quantifizieren könnten. Es ist jedoch häufig schwierig bzw. in vielen Fällen bisher nicht gelungen, Antikörper zu gewinnen, die einerseits exklusiv die aktivierte Form eines Faktors erkennen. Dies ist jedoch in den beschriebenen Präparaten notwendig, da ein Gemisch aus Zymogen und aktivierter Form vorliegt. Andererseits dürfen vorliegende Protease-Inhibitor-Komplexe nicht miterfaßt werden, die die Ergebnisse einer quantitativen Bestimmung der freien, biologisch aktiven Form, verfälschen würden. Diese Komplexe können während der Reinigung des Proteins entstehen bzw. durch gezielt zugesetzte Inhibitoren. Entsprechend ist es Ziel, geeignete Testsysteme bereitzustellen, um diese aktiven Faktoren/Proteasen, besonders die Faktoren VIIa, F IXa, F Xa, F XIa, F XIIa, Kallikrein und APC und Proteasen des Komplementsystemes, quantitativ zu erfassen. Für freies Thrombin mit Hilfe des Thrombin-Antithrombin III-Komplexes in Gerinnungspräparaten wurde ein homogenes Testverfahren in flüssiger Phase beschrieben (Köhler et al., Thromb. Res. 1990; 60: 63-70).

In der vorliegenden Erfindung wird dem Präparat bzw. Konzentrat ein Enzym-Protease-Bindungspartner, bevorzugt ein Inhibitor, zugesetzt, der von einem Antikörper gebunden werden kann, entsprechend inkubiert und die generierten Enzym-/Protease-Inhibitor-Komplexe per Festphasen-ELISA (Enzym-linked-immunosorbent Assay) quantitativ erfaßt.

Als Protease-Inhibitoren kommen bekannte Bindungspartner in Frage, wie Antithrombin III (AT III), α2-Makroglobulin, α1-Antitrypsin, α2-Antiplasmin, Inter-α-Trypsininhibitor, C1-Inhibitor, Protein C-Inhibitor etc., bevorzugt AT III, gegebenenfalls in Gegenwart von Heparin, und α1-Antitrypsin. AT III stellt den wichtigsten physiologischen Regulator/Inhibitor des Gerinnungssystemes dar, der mit aktivierten Faktoren mit unterschiedlicher Affinität/Geschwindigkeit reagiert und einen Komplex bildet. Während z.B. die Reaktion mit Thrombin relativ schnell abläuft und bekannterweise durch Heparin stark beschleunigt wird, findet eine Interaktion mit z.B. FVIIa bei Raumtemperatur oder 37°C nur sehr langsam statt bzw. erst wenn FVIIa im Komplex mit Thromboplastin vorliegt. Eine andere Möglichkeit wurde von Godal et al. (Thromb. Res. 1974; 5: 773-775) beschrieben, in der FVIIa im Kalten für mehrere Stunden mit ATIII/Heparin inkubiert wird und anschließend der restliche gerinnungsaktive FVIIa in einem entsprechenden Aktivitätstest bestimmt wird.

Dem gegenüber wird ein Gewinn an Spezifität und Sensitivität erreicht, wenn die gebildeten Protease-Inhibitor-Komplexe mit Hilfe eines immunologischen Testes quantifiziert werden. Entsprechend werden polyklonale (PAK) oder monoklonale (MAK) Antikörper oder deren F(ab')- oder F(ab)- Fragmente, vorzugsweise ein monoklonaler AK bzw. dessen Fab, nach bekannten Methoden an die Festphase gekoppelt/adsorbiert, die Standard- bzw-Probenlösung damit inkubiert, die Festphase gewaschen und die gebundenen Komplexe mit einem polyklonalen oder monoklonalen AK bzw. Fab gegen den Komplexpartner (Protease oder Inhibitor), inkubiert. Dieser AK ist z.B. mit einem Enzym konjugiert, z.B. POD. Nach Zugabe eines entsprechenden Enzymsubstrates wird der Substratumsatz gemessen und die Konzentration des gebundenen Komplexes mittels einer Standardkurve ermittelt. Die Konzentration der frei in dem Präparat vorliegenden Protease kann entsprechend errechnet werden.
Liegt bereits vor Zugabe einer entsprechend hohen Konzentration des Inhibitors (zur vollständigen Überführung der freien Protease in Komplexe) in dem Konzentrat eine limitierte Menge an Protease-Inhibitor-Komplexen vor (z.B. durch die Präparation bedingt), so wird der Kontrollwert (Präparateverdünnung ohne zusätzlich zugesetzten Inhibitor) vom Probenwert subtrahiert und so die Konzentration der freien, aktivierten Protease errechnet.

In einer bevorzugten Ausführung (A) wird der Inhibitor nach bekannten Methoden direkt an die Festphase gekoppelt bzw. mittels eines an die Festphase gekoppelten Antikörpers (gegen den Inhibitor gerichtet) gebunden und somit den freien in Lösung befindlichen Proteasen präsentiert. Nach Inkubation und Ausbildung der nunmehr immobilisierten Inhibitor-Protease-Komplexe, wird die Festphase gewaschen und die Komplexe mittels eines geeigneten (konjugierten) Antikörpers gegen die Protease oder gegen ein Neoepitop des Komplexes (bzw. mittels eines gegen diese Antikörper gerichteten konjugierten Antikörpers) detektiert.
Gegebenenfalls kann der detektierende Antikörper/Fab bereits während der Inkubation der Festphase mit der Probenlösung anwesend sein.

In einer weiteren bevorzugten Ausführung (B) wird ein für das zu detektierende Enzym/Protease geeigneter Antikörper/Fab an die Festphase gebunden (oder ein gegen diese Antikörper gerichteter Antikörper, z.B. Ziege-anti-Maus, der den spezifischen Antikörper präsentiert). Die das freie Enzym/Protease enthaltende Lösung wird mit der Festphase in Kontakt gebracht und inkubiert. Nach Bindung sowohl der freien, aktivierten Protease (als auch in Lösung befindlicher nicht-aktivierter Enzyme/Proteasen) wird die Festphase gewaschen und mit einer Lösung, die den entsprechenden Inhibitor enthält inkubiert. Nach dem Waschen der Festphase werden die Komplexe mit Hilfe eines den Inhibitor bindenden bzw. ein Neoepitop des Komplexes bindenden konjugierten Antikörpers detektiert. Ebenso kann der die aktivierte, freie Protease enthaltenden Lösung der Inhibitor zuvor (und Inkubation) bzw. während der Inkubation mit der Kontaktphase zugesetzt werden.
Gegebenfalls kann der detektierende Antikörper/Fab bereits während der Inkubation der Festphase mit dem Inhibitor anwesend sein.

Je nach Fragestellung finden die o.g. Ausführungen Verwendung: Während in Ausführung (A) durch den immobilisierten Bindungspartner (hier Inhibitor) exklusiv aktivierte Proteasen gebunden werden und so Spuren dieser nicht-komplexierten Moleküle nachgewiesen werden können, bietet Ausführung (B) zusätzlich die Möglichkeit, die potentielle Präsenz limitierter Enzym-/Protease-Inhibitor-Komplexe bereits vor Zugabe des Inhibitors, zu erfassen. Die parallele Untersuchung der Probenlösung (+/- Inhibitor) und anschließende Subtraktion ((+)Inhibitor minus
(-)Inhibitor) ermöglicht so den Erhalt beider Informationen.

Bevorzugte Paarungen von Bindungspartnern zur Bestimmung aktivierter Faktoren sind
- AT III, plus ggf. Heparin:: F VII a, F IX a, F X a, F XI a, F XII a
- C1-Inaktivator:: F XI a, Kallikrein, C1r
- α₁ Antitrypsin:: aktiviertes Protein C, Elastase, andere Serinproteasen
- Protein C-Inhibitor:: aktiviertes Protein C

### Beispiel:

### Quantifizierung von FIXa-AT III- Komplex (Kx)

### Material:

Mikrotiterplatten wurden mit monoklonalen Antikörpern (mAb, 10µg/ml) gegen FIX/FIXa beschichtet und gewaschen. Zur Detektion von FIXa-ATIII-Kx wurden mit Peroxidase (POD)-konjugierte mAb gegen ATIII verwendet. AT III (Kybernin®), mAb gegen FIX und gegen ATIII stammten von der Behringwerke AG (Deutschland). Heparin (Liquemin®) war von der Hoffmann-La-Roche GmbH (Deutschland). Die Faktoren IX und IXa wurden von Calbiochem (USA) und Serbio (Frankreich) gekauft Das chromogene Substrat S-2765 stammte von Chromogenix AB (Schweden).

### Herstellung eines FIXa-ATIII-Kx Standards:

500 µl einer FIXa (2 µg/ml) enthaltenden Pufferlösung wurden mit dem gleichen Volumen einer den 10fachen molaren Überschuß enthaltenden Lösung an ATIII/Heparin (Verdünnung aus 10 IE/ml ATIII / 20 IE/ml Heparin) für 90 min bei Raumtemperatur (RT) inkubiert. Die vollständige Komplexbildung von FIXa mit ATIII wurde durch photometrische Bestimmung der amidolytischen FIXa-Aktivität mittels S-2765 gegenüber dem Kontrollansatz (ohne ATIII) verfolgt. Nach der o.g. Inkubationszeit war keine Aktivität im FIXa/ATIII/Heparin enthaltenden Ansatz mehr nachweisbar - FIXa war vollständig in den FIXa-ATIII-Kx überführt worden.

### Erstellung einer FIXa-ATIII-Kx Standardkurve:

Aliquots der FIXa-ATIII-Kx Standardlösung (1,0 µg/ml) wurden schrittweise bis zu 1250fach verdünnt und zur Erstellung einer Standardkurve verwendet. In die Vertiefungen der mit anti-FIX-mAb beschichteten Mikrotiterplatten wurden je 50 µl eines Probenpuffers vorgelegt und je 50 µl der Kx-Lösungen dazugegeben.

Nach 1 Stunde Inkubation bei 37°C wurde die Festphase dreimal gewaschen und jeweils 100 µl der anti-ATIII-mAb-POD entahltenden Lösung zugesetzt. Wiederum nach einstündiger Inkubation bei 37°C wurden die Platten dreimal gewaschen; In jede Vertiefung wurden je 100 µl POD-Substratlösung (O-Phenylendiamin-Hydrochlorid) pipettiert, die Platten bei RT für 20 min (im Dunkeln) inkubiert, die Reaktion gestoppt und die OD₄₉₂ gemessen:

| **FIXa-ATIII-KX (ng/ml)** | **OD**_{**492**} **(Mittelwert aus Doppelbestimmungen)** |
|---|---|
| 0,8 | 0,027 |
| 1,6 | 0,028 |
| ∼3,1 | 0,043 |
| 6,25 | 0,09 |
| 12,5 | 0,13 |
| 25 | 0,24 |
| 50 | 0,42 |
| 100 | 0,71 |
| 125 | 0,86 |
| 250 | 1,34 |
| 500 | 1,98 |

Entsprechend ergab sich ein linearer Meßbereich (bei Auftragung log Konzentration Kx versus log OD₄₉₂) von mindestens 1,5 bis 150 ng/ml.

### Quantifizierung von FIXa-ATIII-Kx in FIX/FIXa-Gemischen:

Folgende Mischungen aus FIX, FIXa und ATIII/Heparin wurden hergestellt, für 90 min bei RT inkubiert und als Probe wie oben beschrieben behandelt:

| | | Endkonzentration im Ansatz |
|---|---|---|
| 1. | 40µl FIX (50 ng/ml) | 20 ng/ml |
| | 40µl FIXa (150 ng/ml) | 60 ng/ml |
| | 20µl ATIII/HEp (5 E/ml) | 1 E/ml |
| 2. | 40 µl FIX (100 ng/ml) | 40 ng/ml |
| | 40 µl FIXa (50 ng/ml) | 20 ng/ml |
| | 20 µl ATIII/Hep (5 E/ml) | 1 E/ml |
| 3. | 40 µl FIX (450 ng/ml) | 180 ng/ml |
| | 40 µl FIXa (50 ng/ml) | 20 ng/ml |
| | 20 µl ATIII/Hep (5 E/ml) | 1 E/ml |

Kontrollansätze enthaltend die Faktoren IX, IXa und ATIII/Hep jeweils separat wurden in den unter 1. bis 3. angegebenen Konzentrationen mitgeführt. Weder FIX, FIXa noch ATIII/Hep zeigten eine Reaktion in diesem Testsystem. Dies galt ebenso für das Gemisch FIX/FIXa (ohne nachfolgende Inkubation mit ATIII/Hep) und stellte somit die Selektivität für den FIXa-ATIII-Kx sicher.

Entstandener FIXa-ATIII-Kx wurde entsprechend der o.g. Standardkurve quantifiziert. Der Tabelle sind die Mittelwerte der Ergebnisse getesteter Verdünnungen zu entnehmen.

| Ansatz Nr. (xfach) | FIXa-ATIII-Kx (ng/ml) | Wiederfindung (%) | Verdünnungen |
|---|---|---|---|
| 1 | 58,6 | 98 | 2/4/8/16/32 |
| 2 | 25 | 125 | 4 |
| 3 | 24 | 120 | 1/2 |

## Patentansprüche

1. Heterogenes immunchemisches Verfahren zum Nachweis und zur Bestimmung von aktivierten Enzymen in einer Probe, welches folgende Schritte einschließt:
a) Inkubation der Probe mit einem ersten, für den aktivierten Zustand des Enzyms spezifischen, Bindungspartner,
b) Nachweis der Bildung des Enzym-Bindungspartner Komplexes durch Reaktion des Enzym-Bindungspartner Komplexes mit einem zweiten spezifischen Bindungspartner, der spezifisch mit dem für den aktivierten Zustand des Enzyms spezifischen ersten Bindungspartner reagiert und direkt oder indirekt mit einer signalerzeugenden Markierung versehen ist.

2. Verfahren nach Anspruch 1, dadurch gekennzeichnet, daß der Enzym-Bindungspartner Komplex immobilisiert wird.

3. Verfahren nach Anspruch 2, dadurch gekennzeichnet, daß die Immobilisierung durch den ersten spezifischen Bindungspartner, der an eine Festphase gebunden ist, erfolgt.

4. Verfahren nach Anspruch 3, dadurch gekennzeichnet, daß der immobilisierte Enzym-Bindungspartner Komplex durch einen für die Enzymkomponente oder einen für den Enzym-Bindungspartner Komplex spezifischen Antikörper, der direkt oder indirekt mit einer signalerzeugenden Markierung versehen ist, nachgewiesen wird.

5. Verfahren nach Anspruch 1, dadurch gekennzeichnet, daß das aktivierte Enzym durch einen für das Enzym spezifischen Antikörper, der an eine Festphase gebunden ist, immobilisiert wird, und das immobilisierte aktivierte Enzyme mit einem einem für den aktivierten Zustand des Enzyms spezifischen Bindungspartner, der direkt oder indirekt mit einer signalerzeugenden Markierung versehen ist, inkubiert wird.

6. Verfahren nach einem der Ansprüche 1 bis 5, dadurch gekennzeichnet, daß das aktivierte Enzym ein aktivierter Gerinnungsfaktor ist.

7. Verfahren nach einem der Ansprüche 1 bis 6, dadurch gekennzeichnet, daß die signalerzeugenden Markierung eine chemilumineszente oder fluorogene Markierung ist.

8. Verfahren nach einem der Ansprüche 1 bis 7, dadurch gekennzeichnet, daß die signalerzeugenden Markierung ein Enzym, vorzugsweise Meerretich Peroxidase ist.

9. Verwendung eines Verfahrens nach einem der Ansprüche 1 bis 8 zur Bestimmung von aktivierten Gerinnungsfaktoren in Gemischen aus aktivierten und nicht-aktivierten Faktoren.
